# EUROPEAN PATENT SPECIFICATION

(11) **EP 1 480 703 B1**
(45) Date of publication and mention of the grant of the patent: **19.08.2009**
(21) Application number: 03708071.0
(22) Date of filing: 04.03.2003
(51) Int. Cl.: A61M 25/00, A61B 5/107

(54) **A CATHETER**
KATHETER
CATHETER

(30) Priority: 05.03.2002 GB 0205109
(43) Date of publication of application: 01.12.2004
(73) Proprietor: Thermocore Medical Systems N.V., 9820 Merelbeke, Gent (BE)
(72) Inventor: DIAMANTOPOULOS, Leonidas, B-3140 Keerbergen (BE)
(74) Representative: Finnie, Peter John
(86) International application number: PCT/EP2003/000778
(87) International publication number: WO 2003/074114

(56) References cited:
- EP-A- 1 016 374
- DE-A- 3 617 953
- DE-A- 4 227 367
- US-A- 5 010 892
- US-A1- 2001 039 388

## Description

### Background to the Invention

The human vascular system may suffer from a number of problems. These may broadly be characterised as cardiovascular and peripheral vascular disease. Among the types of disease, atherosclerosis is a particular problem. Atherosclerotic plaque can develop in a patient's cardiovascular system. The plaque can be quite extensive and occlude a substantial length of the vessel. Additionally, the plaque may be inflamed and unstable, such plaque being subject to rupture, erosion or ulceration which can cause the patient to experience a myocardial infarction, thrombosis or other traumatic and unwanted effects.

The study of the vascular wall has proven to be of incomparable value for the percutaneous study for the majority of cardiac diseases. Several techniques have been developed for studying vascular tissue. However, existing methods based on intravascular ultrasound give limited morphological information concerning the tissue characterisation of the arterial wall. Other methods include the measurement of various parameters such as blood pressure, flow velocity, temperature, impedance and the like. These techniques provide poor, or no information about the composition of the vascular tissue. In particular, the above techniques do not provide selective information about the different tissues which make up the vascular wall.

There is a need to produce a method which can be used to detect the composition of the vascular tissue and to provide anatomical and morphological data, thereby yielding information about the quality of the vascular tissue. Analysis of the vascular wall composition can be used to detect early atherosclerosis and other diseases and adverse conditions affecting the vascular tissue, thus rendering the possibility of early treatment of the condition. This allows the possibility of prevention, rather than just cure of such conditions.

EP-A-1016374 discloses an arthroscopic instrument for measuring the distance between two points internal to a body of a patient.

US-A-5, 010, 892 discloses a body-lumen measuring instrument for insertion into a body passageway in order to determine the interior lumen diameter and/or axial length of the passageway at a predetermined location.

### Summary of the Invention

According to a first aspect of the present invention, there is provided a catheter according to claim 1.

According to a second aspect of the present invention, a catheter system comprises a catheter in accordance with the first aspect of the present invention, in combination with a signal processing system electrically coupled to the detector, which is adapted to detect changes in the signal of the detector.

The first aspect of the present invention provides a catheter comprising a resiliently biased projection comprising one plate of a variable capacitor, wherein the capacitance varies as a function of radial displacement of the resiliently biased projection. Thus, a signal processing system electrically coupled to the variable capacitor plate may be adapted to detect changes in the capacitance of the variable capacitor. This affords a method of studying the physiology and/or morphology of a vessel wall by detecting capacitance variations between capacitor plates inserted into the vascular tissue.

The present invention allows dimensional characteristics of the vascular tissue to be determined. For example, the cross-section of a vascular lumen can be measured by measuring the capacitance between the plates of the variable capacitor and relating this to the distance between the plates. If the position of one of the plates in the lumen is known, for example, being positioned against one wall of the vessel, the resiliently biased projection, comprising the other plate of the variable capacitor, can bias itself against the opposing wall. The measured capacitance is directly proportional to the distance between the plates.

One of the advantages of the present invention is that a direct contact method of evaluating the vascular dimensions may be employed. In particular, this has the advantage of employing othertypes of direct measurement, for example, physiological parameters such as temperature of the vascular tissue, and integrate these with the dimensional characteristics of the vascular tissue. This can provide an enhanced "picture" of a portion of vascular tissue. For example, it has been reported that unstable and inflamed plaque can cause the temperature of the artery wall to elevate up to 2.5°C. The present device and method allow the user to establish that not only an inflammation of the vessel is present, possibly indicating stenosis of the vascular tissue, but also that the temperature in the same region is elevated compared to surrounding, non-inflamed tissue. This allows the identity and prioritisation of treatment for unstable plaque.

At least one of the plates of the variable capacitor are attached to the resiliently biased projection or form an integral part thereof. Preferably, pairs of capacitor plates are attached to, or are integrally formed with pairs of opposing resiliently biased projections. Most preferably, at least one plate of the variable capacitor is formed integrally with the resiliently biased projection. Preferably, at least a portion of the variable capacitor forms a substantially flat surface. The variable capacitor is preferably constructed from metal plate, metal foil or a metal film deposited on a substrate. Preferred metals include nickel, titanium, gold, steel, silver and alloys thereof. The electrolytic capacitance created between the plates uses the blood as dielectric. Blood has a high dielectric constant e (serum plasma has approx. e = 200, measured at 1MHz). Thus, with a plate separation of 5mm and a plate area of 1mm² each, capacitance should be C= 8.85 x 10⁻¹² x 200 x 0.000001/0.005 = 0.354 x 10⁻¹² Farads. At a plate separation of 1mm, C= 8.85 x 10⁻¹² x 200 x 0.000001/0.001 = 1.77x 10⁻¹² Farads.

The physiology and/or morphology of a vessel wall is investigated preferably using the frequency shifting of an oscillator due to capacitance variations of a capacitor formed between plates carried by a catheter. Changes in capacitance presented by this variable capacitor are detected by the frequency shifting of an associated variable frequency oscillator which is mixed with the output of a fixed frequency oscillator.

Preferably, the signal processing system according to the second aspect, comprises one or more variable frequency oscillators, the output frequency of which is frequency shifted in dependence on the capacitance presented by a respective variable capacitor of the catheter.

In a preferred example, the signal processing system comprises a first oscillator, the output frequency of which is dependent on the capacitance presented by a respective variable capacitor of the catheter, and a second oscillator, the output frequency of which is fixed, and a frequency mixer which receives the signal outputs of the first and second oscillators and generates a difference frequency signal.

Preferably, the variations in capacitance are detected by the frequency shifting of an oscillator.

The catheter of the present invention is particularly useful for intravascular studies, but equally can be used in other organs or cavities for studying their morphological characteristics and wall composition. Sophisticated computer processing of data can provide information on vascular wall composition and morphology that is hitherto unavailable.

A particular advantage of using variable capacitors is that these methods are particularly sensitive and high resolution of the vessel walls can be obtained. Angstrom changes in distance can result in changes in frequency in the order of KHz.

Such a highly sensitive technique enables the device of the present invention to distinguish between the systolic and diastolic diameters of the blood vessel. Consequently, this enables the user to measure parameters such as the elastic index of the blood vessels. This is particularly useful as it provides information about the physiology of the vessels being studied. For example, a stenosed or calcified region of blood vessel is generally less elastic than a healthy region.

One or more detectors, preferably 2 to 10 detectors, more preferably 2 to 6 detectors may be utilised in the present invention. Preferably, each detector is mounted on a separate projection. In a particularly preferred example, four projections, each having a single detector mounted thereon, are provided.

Generally, the catheter of the present invention comprises a plurality of co-axial lumen. Preferably, the catheter comprises a central lumen adapted to be mounted on a standard angioplasty guide wire suitable for vascular intervention. The apparatus is preferably based on the rapid-exchange or the monorail system, although over-the-wire techniques are also envisaged. Preferably, outside the central lumen is located an intermediate lumen. Preferably, outside the intermediate lumen is mounted an external lumen, hereinafter referred to as a sheath. Preferably, at the distal tip of the apparatus is a guide member. Other lumen may be present and all the lumen may house components within themselves or between adjacent lumen.

The projection is preferably mounted on the central or intermediate lumen but may be attached to any lumen inside the sheath.

The central lumen may be formed from the standard catheter lumen materials, for example, nylon, FEP, polyurethane, polyethylene and nitinol and mixtures thereof.

The intermediate lumen and the sheath are generally constructed from, but individually selected from, the standard catheter lumen materials discussed above.

The sheath is adapted to fit over the adjacent lumen housed inside the sheath and should be able to move relative to the adjacent lumen under the control of a remote device.

Preferably, the central and intermediate lumen are bound to one another and are not moveable relative to one another.

Preferably, the flexible body of the catheter has a longitudinal axis and at least part of the projections are extensible radially from the longitudinal axis of the body. Generally, the projections have an elongate shape, preferably having dimensions in the range of 2 mm to 15 mm, more preferably 3 to 7 mm in length. The projections preferably have a caliper of 0.3 mm to 5 mm, more preferably 0.5 mm to 3 mm.

A first end of the projection is preferably attached to the body, preferably the intermediate and/or the central lumen, while a second end preferably comprises one or more sensors. The second end is preferably free, ie, not attached to any of the lumen, and is adapted to be radially movable away from the central lumen.

The projections utilised in the present invention preferably comprise sensors, preferably temperature sensors.

One or more sensors, preferably 2 to 10 sensors, more preferably 2 to 6 sensors may be utilised in the present invention. Preferably, each sensor is mounted on a separate projection. In a particularly preferred example, four projections, each having a single sensor mounted thereon, are provided.

Where more than one projection is provided, each projection is preferably independently biased. Thus, each projection can follow the vessel morphology independent of the other projections.

The sensors are preferably located on an outer face of the projection, relative the central lumen, ie., facing the vascular tissue in use. Each sensor should preferably be located toward, or at the distal tip of the projection.

The capacitor plate(s) is/are preferably located on the inner face of the projection, relative to the central lumen. In a particularly preferred example, four projections are provided, and each comprising a capacitor plate.

The projections need not be mounted in substantially the same circumferential plane of the catheter body, but this configuration is preferred.

It is also possible to provide projections having different lengths. This allows a better assessment of the 3D location of the projections to be provided while using a 2D imaging technique.

The projection preferably comprises a superelastic material. Superelasticity refers to the ability of certain metals to undergo large elastic deformation. Such compounds favorably exhibit features such as biocompatibility, kink resistance, constancy of stress, physiological compatibility, shape-memory deployment, dynamic interference, and fatigue resistance.

A large number of super-elastic materials may be utilised, particularly binary Ni-Ti with between 50 and 60 atomic percent nickel. While many metals exhibit superelastic effects, Ni-Ti-based alloys appear to be best suited for deployment in the human body due to them being chemically and biologically compatible.

Preferably, the projection, when not restrained will adopt a deployed configuration in which a free end of the projection is extended away from the central lumen. In this deployed configuration, the projection is resiliently biased against the vascular wall in use, thus initiating contact between the sensor and said wall. This achieves an adequate contact with the vascular wall, without substantially compromising blood flow.

Preferably, a heat shrink wrapping is applied over at least a portion of the length of the projection. A heat shrink material is generally a polymeric material capable of being reduced in size upon application of heat. These are generally used in the form of a tube. Suitable materials include polyesters, PVC, polyolefins, PTFE and the like. The preferred material is a polyester.

Preferably, the heat shrink material covers the detector and isolates it from the body of the subject of the interventional surgery. Preferably, the heat shrink material additionally covers the sensor.

In accordance with a particularly preferred example of the first aspect of the invention, the resiliently biased, projection when restrained, adopts a substantially straight shape, which lies substantially parallel to the longitudinal axis of the catheter body. In the deployed configuration, the projection adopts an arcuate shape along at least part of its length. In this embodiment, the gradient of the arcuate portion of the projection, with respect to the longitudinal axis of the catheter, increases as a function of distance along the projection from the end attached to the catheter body. Thus, the free end of the projection bends away from the catheter body. This particular embodiment allows the free end of the projections to more accurately and consistently follow the morphology of the vascular tissue. A stenosis usually involves a section of the wall being inflamed and thus protruding into the lumen of the blood vessel. Alternatively, a calcified plaque may have an irregular surface leading to it protruding into the lumen. Where an arcuate deployed projection is employed, the arc allows the tip of the projection to "reach around" to the trailing edge of a stenosed region as the catheter is moved along the vascular tissue. The arcuate nature of the projections also allows the temperature sensors, where present, to be located more directly and in closer contact to the vessel wall as well as providing a more accurate morphological resolution of the vessel wall. The maximum gradient of the projection, with respect to the longitudinal axis of the catheter body is preferably less than 90°, more preferably less than 75°, more preferably less than 60°. In this particular embodiment, the arc of the projection preferably provides maximum possible contact angle between the projection and the vessel wall of less than 90°, more preferably less than 75°, more preferably less than 60°. This angle, while having a maximum deviation of less than 90°, is variable as a consequence of the compliant nature of the biased projection. This allows the projection to follow the vascular morphology.

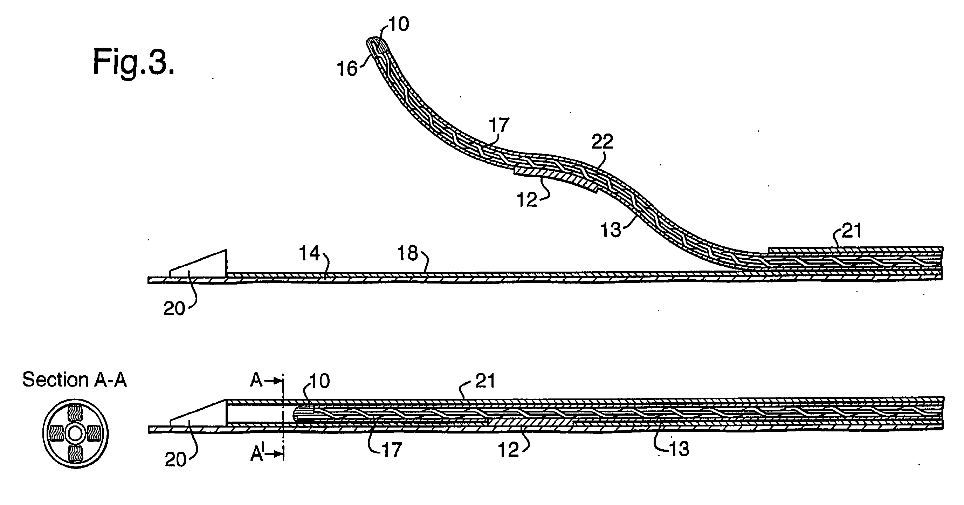

Where a projection having an arcuate portion is provided, there may also be substantially straight portions of the projection along its length. A particularly preferred example provides a resiliently biased projection having a substantially straight portion which bears the detector, in particular, a capacitance plate. It would be advantageous to produce capacitance plates which remain substantially parallel even during displacement of the resiliently biased projections. This may be achieved using a sinusoidal shaped projection, preferably a flattened sinusoidal shape having about 1.5 wavelengths of a sinusoidal wave. This structure could be described as an "extended S" shape. An example is shown in figure 3. This shape provides an arcuate portion which enables good contact with the vessel wall, as described above. It also provides a section which is flattened (in this case towards the middle of the projection) upon which the detector may be mounted.

In a particularly preferred example, the capacitor comprises two or more resiliently biased projections, each of which may comprise one plate of the same variable capacitor. Thus, a preferred example is a catheter comprising two resiliently biased arms, each of which comprise one plate of the same variable capacitor. Consequently, the resiliently biased projections may be positioned on opposed sides of the catheter at, for example, 180° intervals. This allows the resiliently biased projections to bend away from the capacitor body and to contact opposed vascular walls. As the resiliently biased projections are conformal to the morphology of the vascular tissue, a cross-section, or a series of cross-sections of vascular morphology may be measured by relating the change in capacitance to the change in distance between the plates of the variable capacitor as the projections contact the vascular wall. It should be understood that any number of capacitor plate pairs may be used in the present invention. These may each be mounted on independently biased projections. Where, for example, 4 plates are provided on 4 projections, the projections may be positioned at substantially 90° intervals. The opposed (180° separation) plates preferably bear paired capacitor plates. This allows an eccentric assessment of vessel dimensions rather than just concentric.

In another particularly preferred example of the first aspect of the present invention, a catheter is provided comprising two or more resiliently biased projections each comprising one plate of different variable capacitors, and at least one fixed plate comprising the other half of at least one of the variable capacitor plates on a resiliently biased projection. This example allows the fixed plate to be provided, preferably mounted on, or integral with, the catheter body. The fixed plate is associated with at least one of the plates mounted on one of the resiliently biased projections. Thus, the capacitance can be measured between the projection-mounted plate and the fixed plate. The term "fixed" is intended to distinguish from the projections which are moveable relative to the catheter body. "Fixed" is not intended to imply that the plate is stationary when in the vessel. In this particular example, the same fixed plate or another fixed plate may be associated with the variable capacitor plate mounted on the second projection.

In an alternative example, the projection may be mounted to achieve a similar resiliently biased effect. For example, one method of achieving this would be to mount the projection on a spring, preferably a micro-spring, such that when unrestrained, the projection is extended against the vascular wall as discussed above.

The sensors may be any form of temperature sensor and are preferably selected from thermistors, thermocouples, infra red sensors and the like. Preferably, the sensors are thermistors. These are preferably semi-conductor materials having an electrical impedance in the range of 1-50 KΩ. Such thermistors prove extremely reliable regarding the relation between the temperature changes and resistance changes.

Preferably, the catheter comprises a radiopaque marker which aids in the location of the device by fluoroscopy during interventional surgery. More preferably, at least one detector includes a marker so that it is discemible via fluoroscopy. Most preferably, individual detectors include different marker types, so that using fluoroscopy, the individual detectors can be identified and their spatial orientation and relative location to a desired part of the vessel wall thus clearly defined.

The detector is preferably attached to an electrical carrier, preferably a wire, which allows the data from the detector to be connected to a remote device to the personal computer. The wire(s) are preferably housed within the sheath and are preferably electrically isolated from the patient. Preferably, the wire(s) are housed between the central lumen and the intermediate lumen, within the outer sheath.

The proximal section of the catheter incorporates a connector for coupling the detected signals to a remote device such as a personal computer. These signals are transmitted along the wire(s) from the detector. The wire(s) are preferably housed within the sheath and are preferably electrically isolated from the patient. Preferably, the wire(s) are housed between the central lumen and the intermediate lumen, within the outer sheath.

Where sensors are provided, these may be similarly linked via an electrical carrier to a remote device.

In a particularly preferred example, electrical carrier connected to the detector and/or sensor for transmitting data to a remote device is coiled. Preferably, the electrical carrier is coiled around the body of the projection. Such a device is described in our earlier filed European patent application no. 01306599.0 In this embodiment, the electrical connection is coiled to reduce the strain at critical points where it is necessary to maintain a seal, and hence electrical isolation. The coiled nature of the carrier also allows the carrier to act as an inductance coil.

The design is also especially suitable for use with a vascular thermography catheter apparatus of the type described in our earlier filed International patent application no. PCT/EP01/04401.

In a particularly preferred embodiment of the present invention, the catheter may be used in concert with a catheter positioning system in accordance with that disclosed in our co-pending European application No. 01307682.3. This system comprises a guide catheter extension adapted to co-operate with a guide catheter, a catheter positioning device adapted to engage a catheter and guide the catheter within the guide catheter extension, wherein the guide catheter extension further comprises a plurality of engagement means for fixing the relative positions of the guide catheter extension and the positioning device at any one of a number of positions over its length.

This system allows the distance between a guide catheter and a positioning device to be manipulated by the user. Thus the guide catheter may be fixed in position relative to both patient and positioning device, while providing the optimum distance between the effective length of the guide catheter (guide catheter and guide catheter extension) and the points at which the catheter is fixed to the positioning device.

The guide catheter extension is adapted to receive a catheter used in interventional cardiology. Preferably, the body of the guide catheter extension is substantially cylindrical in cross section and has a diameter in the range of 1-15 mm.
Preferably the diameter is in the range of 2-10 mm, more preferably 3-7 mm. Preferably, the length of the guide catheter extension is in the range of 0.1 m to 1 m. More preferably, the length of the guide catheter extension is 0.15-0.5 m.

The body of the guide catheter extension may be formed from standard guide catheter materials. For example nylon, PTFE, polyurethane, polyethylene and nitinol and mixtures thereof may be used. It may also be made from metals such as aluminum, steel and alloys thereof.

The guide catheter extension preferably has a number of points adapted for engagement with the catheter positioning device. Notches, annular indentations, and any other suitable means may be used. Preferably there are 2-200 fixation points, more preferably 5-100, most preferably 10-50 fixation points. These engagement means enable the guide catheter extension to be fixed in place, at selected positions over its length, on the catheter positioning device.

The guide catheter extension comprises a distal and a proximal end. Preferably, the distal end is adapted for engagement with the guide catheter, while the proximal end is adapted for engagement with the catheter positioning device.

There is also provided a guide catheter extension capable of receiving a catheter, comprising a substantially rigid tubular section capable of sealing engagement within a compression fitting of the guide catheter.

Where positioning of the catheter, therefore translational movement within the vascular tissue (therefore also within the guide catheter and guide catheter extension) is required, the arrangement allows the junction between the guide catheter extension and guide catheter to be sealed by tightening the compression fitting, but does not allow the junction to impinge on the catheter within. The seal is preferably achieved by providing a sealing element in the guide catheter extension which forms a low friction, slidable seal with the sheath of the catheter. Thus the catheter is able to be moved and positioned within the apparatus without undue friction being applied to the catheter. This is particularly important as a Y-piece, in addition to being used as the injection point for contrast medium into the patient is also used as a pressure measurement point during the interventional procedure. In order for the pressure of the patient to be reliably measured, the system must be substantially closed, otherwise the pressure will vent at a non closed section. This will lead to loss of pressure, loss of blood, and unreliable pressure readings. However, the present system maintains the pressure of the system as the guide catheter and guide catheter extension junction is sealed and the diameter of the catheter is generally slightly less than the diameter of the internal lumen of the guide catheter extension. Alternatively, the pressure is maintained by providing the above mentioned sealing element in the guide catheter which forms a low friction, slidable seal with the sheath of the catheter.

Most preferably, the distal end of the guide catheter extension is adapted for engagement with a standard Y-piece used in interventional cardiology, having a compression fitting. This substantially prevents loss of blood or fluid at the junction between the guide catheter and the guide catheter extension.

Preferably the distal end of the guide catheter extension comprises a substantially rigid tubular section which is fixed to a flexible section, and which is co-axial therewith. The rigid tubular section may be integrally moulded with the flexible section. Alternatively, it is fixed to the flexible section by any suitable means, for example, glue, soldering, welding and the like.

The catheter positioning device is preferably a type for positioning a catheter and comprises a first lumen mount for holding a first lumen of the catheter, a second lumen mount for holding the guide catheter extension, and a drive mechanism, wherein the first lumen mount is selectively connectable to the drive mechanism for relative movement with respect to the second lumen mount

The second lumen mount preferably includes a bracket, preferably adapted for engagement with the guide catheter extension. The bracket is usually located at one end of an extension arm, while the other end is connected to the body of the positioning device.

The positioning device is preferably a pull-back device which is particularly useful when used in concert with a vascular catheter apparatus according to a first aspect of the present invention. The catheter requires precise positioning and or maneuvering within vascular tissue. The positioning device also allows precise and controllable movement within the vascular tissue. This enables the precise vascular mapping of the vessels morphology.

When the pull-back device is used in concert with the types of vascular catheter according to the present invention, the pull-back device preferably comprises a first lumen mount for holding a first lumen of the catheter, and a second lumen mount for holding a second lumen of the catheter, a third lumen mount for holding a guide catheter extension, and a drive mechanism, wherein each of the first and second lumen mounts is selectively connectable to the drive mechanism for both independent and relative movement with respect to the third lumen mount and to one another to control the configuration of the catheter.

The pull-back device enables a guide catheter and the catheter to be stabily mounted. In particular, the pull-back device enables relative movement between the guide catheter and the catheter but, in use, allows the catheter to move relative to the patient and restrains movement of the guide catheter relative to the patient The pull-back device additionally allows a controlled retraction and positional retention of the associated sheath, thus ensuring atraumatic expansion of the projections on the catheter.

Preferably, the pull-back device comprises a fixed mount for the guide catheter extension, a mount for the sheath and a mount for the combined inner and intermediate lumen. Hereinafter, the guiding catheter extension mount is referred to as mount A, the sheath mount as mount B, and the inner and intermediate lumen mount as mount C.

Mount A preferably has a fixed position during pull-back but may be adjustable. Mount B and C are preferably moveable relative to one another and to mount A. Mount B and C may be motor driven, in particular stepper motor driven. While mount B and C are moveable, they are preferably adapted to enable selective locking in place relative to one another and/or to mount A. Mount C is preferably mounted on the drive mechanism although mount B and C may both be mounted on the drive mechanism.. The drive mechanism enables the catheter to be driven towards or away from the patient via movement of mounts B and/or C.

The interlocking of mount B and C prevents the sheath from moving relative to the lumens housed inside the sheath, thereby ensuring the projections remain in the deployed configuration and engaged with the vascular tissue in the area of interest.

The locking mechanism on the pull-back device includes a restraining mechanism, preferably a stopper rod. This is provided with means for engaging projections within mounts B and/or C. A similar set of projections within the same mounts are used to selectively connect the mounts to the drive rod. These projections may be actuated by a user who can selectively control which of the mounts is locked and which are driven, and the interaction between the mounts.

The drive mechanism is preferably driven by a motor, and preferably gearing is provided along with control and monitoring means.

It is particularly important that substantial occlusion of the vascular tissue is prevented. This is achieved by the present invention as the apparatus in a deployed configuration does not substantially increase its radial cross sectional area beyond the radial cross sectional area of the apparatus in a retracted configuration.

Preferably, the ratio of the area of the cross-sectional profiles of the apparatus in the deployed to retracted configurations is in the range 4:1-1:1, preferably 3:1-1.25:1, more preferably 2.5:1-2:1, most preferably 1.75:1-1.25:1.

The vascular catheter apparatus of the present invention, subsequent to the identification and measurement of vascular tissue, in particular, atherosclerotic plaque, may be used to treat an area identified as being at risk of rupture of said plaque. Treatment may be effected by reinserting the catheter to a predetermined area of the vascular tissue. This reinsertion may be achieved in a controlled manner as the prior morphology measurement scan with the device may be used to produce a morphological map of the vascular tissue. This information may be stored in the remote device and can be used to relocate the area of risk. This procedure requires less contrast media to be infused into the patient than would normally be required in similar vascular interventional procedures as the position of the catheter is known due to the data stored in the remote device. The pull-back device may then, under the control of a user, be used to drive the catheter back to, for example, the starting point of the morphological measurement or any point along the path of the morphological data acquisition, for further morphological or physiological measurements or alternative treatments of the vascular tissue.

For example, the catheter apparatus can then be used to treat the area by any of the usual therapeutic procedures, including localised delivery of a therapeutic agent, delivery of a stent, brachy therapy, ablation of selected tissue etc. Thus the catheter may additionally comprise angioplasty balloons or sleeves.

### Brief Description of the Drawings

Examples of the present invention will now be described in detail with reference to the accompanying drawings, in which:
Figure 1 shows a schematic diagram of a system for conducting vascular catheterisation of a patient;
Figure 2 and 2a shows a side view of the distal end of the catheter of the present invention;
Figure 3 shows a sectioned view of the catheter of the present invention with an extended S-shape profiled projection:
Figure 4 shows the pull-back device in side view;
Figure 5 shows the pull-back device in plan view;
Figure 6 shows a cross-sectional view of the catheter guide extension;
Figure 7 is a flow diagram illustrating the steps involved with conducting intravascular catheterisation of a patient and the associated data capture and image processing; and,
Figure 8 shows an example of a signal processing unit for use with a catheter of the present invention.

### Detailed Description

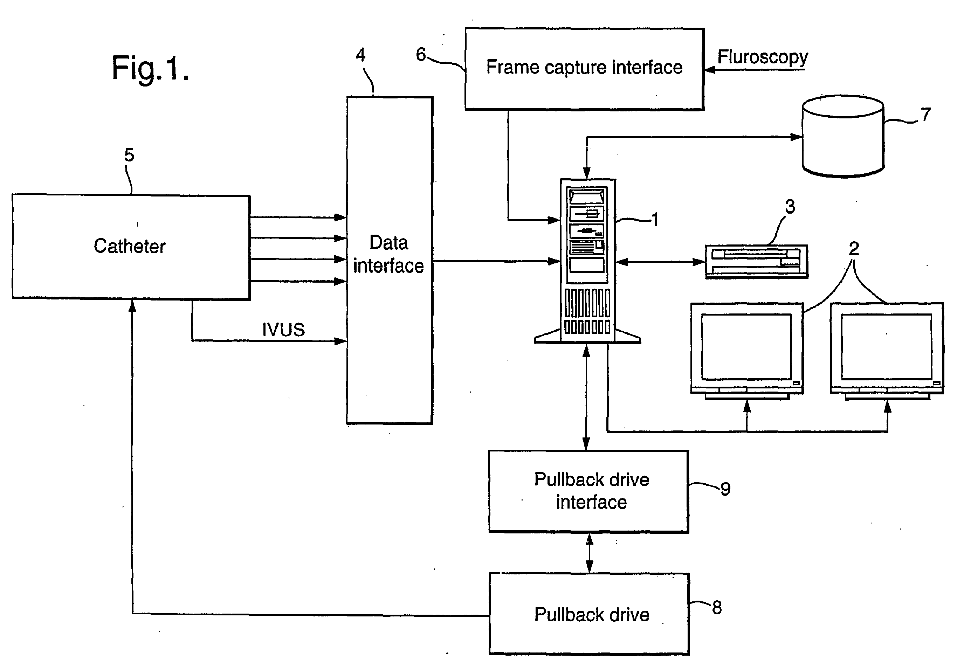

Figure 1 is a schematic diagram of a system for conducting vascular catheterisation of a patient

The system includes a personal computer (PC) 1 that presents a general user interface (GUI) via a number of monitors 2. The user interface system is based on a Microsoft Windows™ platform. Multiple windows may be used to acquire/project data from/to the user. Although not shown, the PC can accept user inputs via a keyboard and mouse, or other pointing device, in the usual manner. The PC includes a number of data stores 7, which may be external, and a CD ROM reader/writer device 3.

The PC is coupled via a data interface 4 to a catheter 5, details of which will be described below. In this example, the catheter 5 transmits four channels (one for each detector) which are received by the data interface 4. An analogue capacitance data signal on each channel is converted to a digital signal using an A/D converter within the data interface 4 at a user configured sampling rate of up to 2.5 KHz. Typically, the sampling rate would be set at around 25 to 50 Hz to reduce the quantity of data acquired.

The data interface 4 includes a multiplexer (not shown) that combines the four digital channels into a single time division multiplexed (TDM) signal. This TDM signal is coupled to the PC over a PCI bus. The data from each channel is written into an area of memory within the data store 7 reserved for that channel where it can subsequently be retrieved for data processing along with the corresponding time sequenced data from other channels and image data from other sources.

The capacitance data from the catheter 5 is introduced to the system software running on the PC using function calls. Capacitance data are input to the software as the frequency at the A/D hardware inputs, and therefore they have to be converted to distance. The frequency changes are first converted to voltage via a frequency to Voltage converter, and then they are driven to the A/D coverter. A detector data convert function handles this process.

This particular system is designed to be used in conjunction with temperature sensing apparatus. The temperature data can be processed in a similar way to the capacitance data, as discussed in the preceding paragraphs.

The system is designed to be used in conjunction with a fluoroscopy x-ray apparatus and therefore includes a video frame capture interface 6 that couples fluoroscopy video data inputs to the PC via a PCI bus. Similarly, it can be used in conjunction with intravascular ultra-sound (IVUS) image data fed from the catheter 5 (when provided with the appropriate hardware). The system software allocates sufficient memory area to the systems memory for this data, taking into account the current system configuration, for example sampling rate, recording time, and video frame size. A memory handle hDib is used to map video data directly through the PCI bus from the video frame capture interface 6 to this allocated area in memory. hDib memory is divided into i equal chunks, each of a size equal to the frame capture interface frame-buffer. Optionally, hDib [i] data can also be mapped to a memory area of a screen-video buffer, giving capability of live preview during recording. Each time the software records an x group of four (or more) capacitance measurements, it prompts for a frame capture at hDib [x]. A user configuration file determines the ratio between capacitance data:fluoroscopy video frame capture.

Whilst in normal circumstances the catheter 5 is inserted manually, it is intended that when performing vascular measurements the catheter 5 is pulled back relative to a predetermined start position using an electro-mechanical pull-back drive 8 coupled to the body of the catheter. The pull-back drive 8 is controlled by the PC via a pull-back drive interface 9. The system software accesses user-defined configuration files to get the necessary information about controlling the systems automatic pull-back interface 9. Data sampling rate, recording duration and preselected retraction rate are taken into consideration for adjusting the pull-back speed.
The software routines control a D/A converter (not shown) that feeds the input of the pull-back interface 9 with an appropriate control voltage. The controlled pull-back process will be described in more detail below.

Capacitance data plotting may be both on-line and/or off-line. In an on-line mode, the monitor presents a capacitance/time-distance graph, where capacitance is continuously plotted as connected dots. In an off-line mode, capacitance data can be loaded from the data store 7 (or other media) and plotted on the screen graph. The user can scroll to different time/temperature locations, while several automated functions may be provided.

The system software is designed to provide basic and advanced image processing functions for the captured fluoroscopy/IVUS video frames, such as filtering and on-screen measurement functions. The user can filter the captured frame to discard unwanted information while focusing on the desired one. There are several auto-filter options as well as manual adjustment of the image curve. In addition, the user can calibrate the system and proceed in performing on-screen measurements of both distances and/or areas. Automatic routines perform quantification of the measurements giving significant information on lesion characteristics.

By using capacitance data and video frame data, the system software uses advanced algorithms based on interpolation and fractal theory to plot a 3D reconstruction of the vessel under measurement. The user can freely move the virtual camera inside the reconstructed vessel in 360°, and/or fly-through the vessel. 2D reconstructions are also provided.

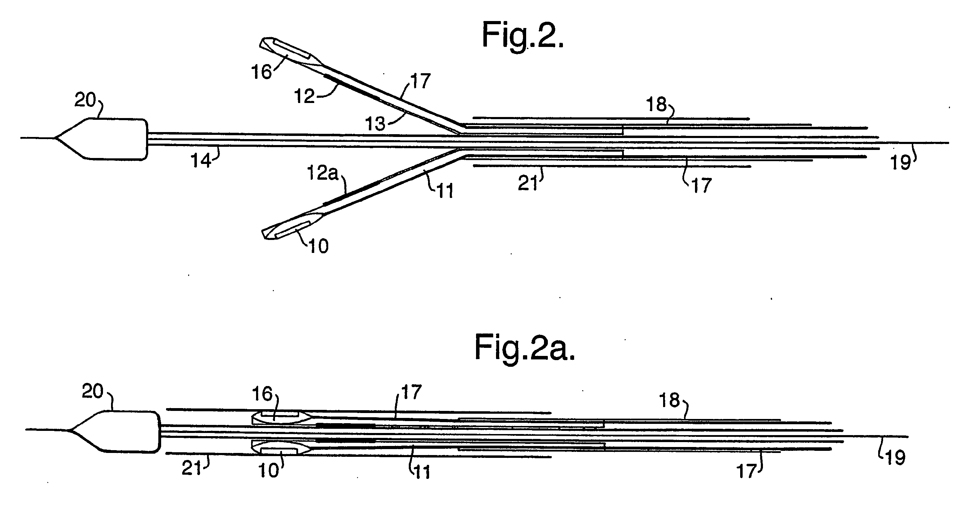

Figure 2 and 2a shows one example of the distal tip of a catheter incorporating sensors 10 mounted circumferentially about a central lumen 14. In this example, four sensors 10. are mounted on resiliently biased projections 11 circumferentially about the central lumen at 90° intervals, although only two sensors are shown here for the sake of clarity.

Variable capacitor plates 12 and 12a are mounted on the side of the face of the projections facing the central lumen 14. In this example, four variable capacitor plates 12 and 12a are mounted on resiliently biased projections 11 circumferentially about the central lumen at 90° intervals, although only two variable capacitor plates, 12 and 12a, are shown here for the sake of charity.

In this example, the opposed plates, 12 and 12a, are a pair of plates making a single variable capacitor.

Each plate 12 and 12a is embedded within a plastics covering, although it could instead be surface mounted. The shape and configuration can be modified to provide different shaped plates, different plate spacings, and different longitudinal coverage for the or each pair of plates.

Each plate 12 is connected to the proximal part of a catheter (not shown) via a respective thin electrical wire 13 carried within the body of the catheter 10 (in the Figure, some electrical wires have been omitted for clarity). Each electrical wire 13 is electrically shielded along its length to avoid interference. As will be described in detail below, each electrical wire 13 connects to an interface forming part of a signal processing system that is used to detect changes in the effective capacitance presented by each pair of plates 12 and 12a. As an alternative, portions of the signal processing system described below can be incorporated within the body of the catheter itself to eliminate interference.

The sensors 10 are NTC thermistors. Such thermistors prove extremely reliable regarding the relation between the temperature changes and resistance changes. An NTC thermistor having a 30 KΩ impedance at 25°C typically maintains linearity between 35°C and 45°C, at a resolution of 0.01 °C - 0.1 °C.

The construction of the thermistors 10 are that of two rectangular plates with a metal alloy oxide in the centre. The thermistor has dimensions in the range of 0.25mm - 5mm, and a caliper less than 1mm.

Each thermistor 10 is permanently attached to the end of each projection 11 by bonding with an thermally conducting epoxy glue 16. Each thermistor 10 is permanently connected to an insulated wire 17, preferably an insulated bifilar wire. The wire 17 has a low impedance and is constructed from nickel and/or copper. This wire provides an electrical connection with the proximal end of the device (not shown).

The projections 11 are mounted on the central lumen 14 and sandwiched between the central lumen 14 and an intermediate lumen 18. The point at which the projections 11 meet the central/intermediate lumen terminus is sealed. This means that the components located between the central and intermediate lumen are electrically isolated from the patient except through the projections. This also means that no air or debris which may find its way into the space between the lumen can be transmitted to the patient.

As shown in Figure 2 and 2a, the catheter is mounted on an angioplasty guide 19 wire which runs through the central lumen 14 and a guide member 20 which defines the distal tip of the catheter.

In use, the apparatus may be actuated between a non-wall-temperature sensing configuration and a temperature sensing configuration. The non-temperature sensing configuration is hereinafter referred to as the retracted configuration. The temperature sensing configuration is hereinafter referred to as the deployed configuration. An example of the deployed configuration is shown in Figure 2. An example of the retracted configuration is shown in Figure 2a.

In the retracted configuration, a sheath 21 encompasses the projections 11 so that they are constrained to lie parallel to the longitudinal axis of the catheter and therefore cannot take up a deployed position. The sheath 21 extends as far as the rear end of the guide member 20 but does not overlap the guide member. This minimises any protrusions from the catheter which could lead to damage of the vascular wall. This is particularly important where a vessel is angulated or there is bifurcation of the vessel. Such features lead to bending of the catheter and would emphasize any protrusions. Hence, in this example the sheath 21 and the guide member 20 present a smooth profile when adjacent to one another in the retracted configuration.

To adopt the deployed configuration, the sheath 21 is withdrawn away from the extreme distal tip i.e., away from the guide member 20, towards the proximal section, to expose the projections 11. When the sheath 21 is withdrawn to the extent shown in Figure 2, the resiliently biased projections 11 take up the deployed configuration. It should be noted that the sheath is controlled from the proximal end of the apparatus and is not shown in its entirety in the Figures.

In the deployed configuration, the sheath 21 is retracted until it is at least level with the mountings for the projections 11 on the intermediate lumen 18 so that it does not impede the movement of the projections.

The projections are made of NiTinol and take on the deployed configuration automatically due to their superelastic properties.

It should be noted that each projection 11 is effectively independent and thus may extend to the vascular wall in the deployed configuration but will not exert high levels of force upon the wall.

An excessive force should not be exerted on the vascular wall. This will vary between one type of vascular wall and another. The apparatus should exert enough force to enable an adequate thermal contact between the sensors 10 and the vascular wall. More particularly, when the catheter is in the deployed configuration, preferably all of the projections 11 are in contact with the vessel wall at any one point in time.

The projections 11 individually extend a certain angle of expansion (r) away from the longitudinal axis of the catheter. In the deployed configuration, r has a value in the range of 15°-70°. However, r is not fixed and varies with the diameter of the vascular tissue being measured due to the flexibility of the projections 11.

Different diameter catheters may be used for different diameters of vascular tissue. However, as it is desirable to minimize the diameter of catheters in all interventional vascular treatments, it is desirable to adapt the length of the projections and/or the angle to which the projections may extend away from the central lumen depending on the dimensions of the vascular tissue being measured rather than increasing catheter body dimensions. Thus, the projections for a large blood vessel, for example 8 mm diameter, will generally require a length of projection in the range of 5 mm to 10 mm. Smaller diameter vascular tissue, for example 2.5 mm diameter, will generally require a length of projection in the range of 2 mm to 6 mm. Typically, the ratio of the area of the cross-sectional profiles of the apparatus in the deployed to retracted configurations is up to 4:1.

The catheter includes a valve system (not shown) allowing the central lumen 14 to be flushed in an adequate way, thus minimising the possibility of air bubbles or debris within the lumen. Such a valve is constructed to enable engagement by a 2 mm, 5 mm, or 10 mm, 6° luer syringe. The catheter may be flushed with a suitable fluid such as saline. When flushing the catheter, fluid should exit via the distal tip of the catheter, indicating proper flushing of the central lumen 14. The proximal section of the catheter (not shown) incorporates a connector for the capacitance and temperature signal transfer to the data interface 4. The connector contains five female plugs to assure proper transmittance of the electrical voltage signals transmitted from the four thermistors 10, and the frequency signals transmitted from the four capacitor plates 12. These signals are transmitted along the wires 17 from the four thermistors 10 and the four wires 13 from the 4 capacitor plates 12. The five female plugs concerned with plates 12 are connected to four detector wires and one common ground. A directional, 5 pin, gold plated, water-resistant connector is used.

Figure 3 shows the deployed configuration projection adopting an arcuate shape along part of its length, with the gradient of the projection, with respect to the longitudinal axis of the catheter, increasing as a function of distance along the projection from the end attached to the catheter body. The projection shown adopts an "extended S" shape. As discussed above, this allows the arcuate portion, at the distal end of the projection, to achieve adequate contact with the vessel wall, while providing a section, towards the middle of the projection, where the capacitor plate is mounted. This section remains relatively parallel to longitudinal axis of the catheter body, even upon radial displacement of the projection.

As shown in the Figure 3, the wire 17 is coiled around the length of the projection 11. This feature has the effect of substantially eliminating strain when the projection 11 flexes. The pitch of the coil is typically arranged to be such that there are 5 to 10 turns over a length of 10 mm. As will be described below, a heat shrink wrapping 22 is applied over the projection 11 to prevent damage to the wire 17 during retraction and replacement of an outer sheath 21. The heat shrink wrapping also provides an additional degree of electrical isolation.

To assemble a projection, a NiTinol arm is first pretreated by placing it in a bending tool and heating to around 700°C to impart a bend(s) in the arm. The NiTinol arm is then held straight in a chuck and a thermistor /bifilar wire assembly is attached to a free end of the arm using a UV cure adhesive. The wire 17 is then spun around the length of the NiTinol arm. Finally, the heat shrink wrapping 22 is placed over the length of the NiTinol arm to a point just beyond that of the thermistor, In this example, the heat shrink wrapping is supplied as a polyester tube that is cut to length. An epoxy resin is then injected into the end of the tube. The assembly is subsequently heat treated to shrink the tube and set the epoxy resin. The heat shrink wrapping is then trimmed back to expose at least part of the epoxy resin coated thermistor, while maintaining electrical isolation of the bifilar wires. After heat treatment, the heat shrink has a wall thickness of around 10µm. The capacitor plate may be attached to the projections prior to encapsulation, or may be attached to the outside of the shrink wrapping and further encapsulated with another shrink wrapping.

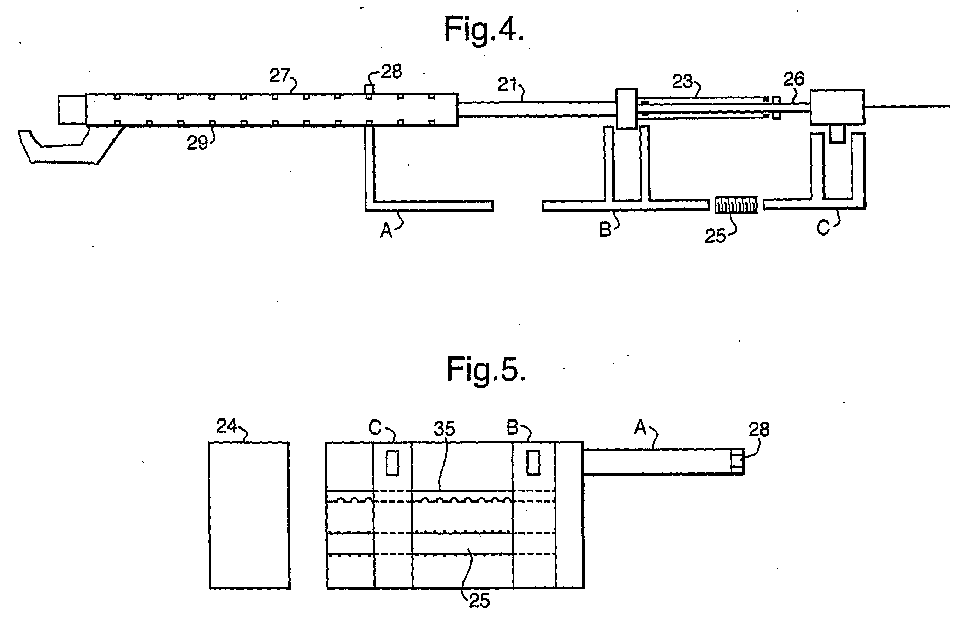

The body of a pull-back device is illustrated in Figures 4 and 5. The proximal section of the catheter described above is constructed to enable remote deployment and retraction of the projections. This is effected via manipulation of the sheath. A two-lumen telescopic construction 23 is used to manipulate the sheath 21 between the retracted and the deployed configuration. One lumen is connected to, or integral with, the outer sheath and can slide over an adjacent lumen which comprises or is connected to one of the lumen housed within the sheath. Rotation of one tube inside the other is prevented by slotting of the lumen or other features on the lumen. Additionally, scaling markings (not shown), may be provided to avoid over-retraction of the tubes.

The pull-back device includes a drive module 24 which includes a motor, gearing system, typically a speed reducer, control and monitoring means, and engagement gear for a driving rod 25. The drive module may be formed separately from the body of the pull-back device so that it may be reused. The body of the pull-back device must be kept sterile and may be formed from a material such as polyurethane. This allows the body to be cheaply and easily produced and may be disposable. Alternatively, or additionally, the pull-back device may be enclosed in a sterile, flexible plastic sheath when in use, so as to maintain sterility.

The pull-back device comprises a driving rod 25, adapted for engagement with an engagement gear of the drive module 24 and mount C. Mounts C and B are adapted to engage the central/intermediate lumen 26 and the sheath lumen 21 respectively. A Mount A is provided which is adapted to engage the guide catheter extension 27. Mount A includes a bracket 28 for connection of mount A to the guide catheter extension fixation points 29. When engaged, mount B may be moved towards C to place the catheter in the open configuration. C may be selectively driven reversibly over a range of travel (usually about 60 mm) suitable for withdrawal of the catheter apparatus over the measured region. The driving rod 25 is a worm-screw type which interacts with the engagement gear of the drive module 24, thus providing a smoothly driven apparatus.

The mounts B and C may individually be locked in position relative to one another or may be selectively unlocked in order to allow movement of the lumen 26, sheath 21 and guide catheter 27 relative to one another.

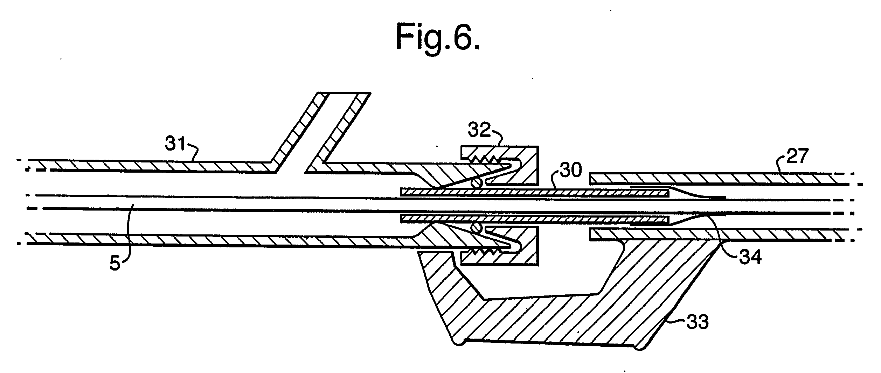

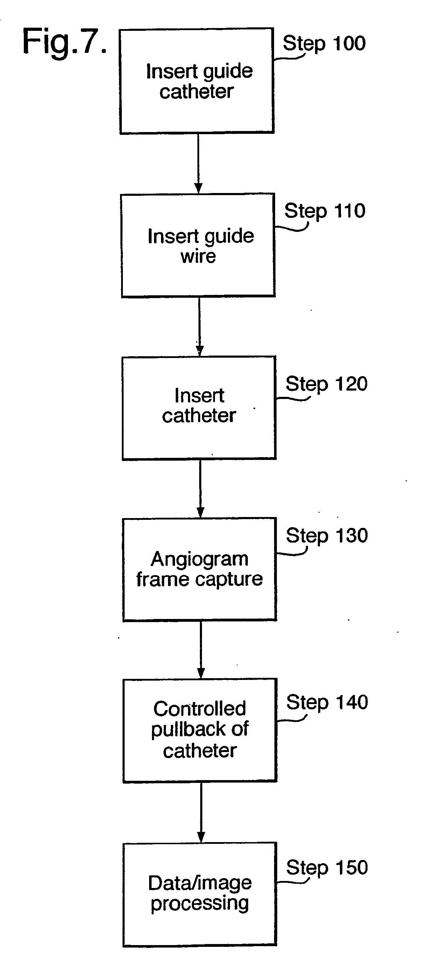

With reference to Figures 6 and 7, in use, the sequence of events begins with the insertion of a guiding catheter into the area of general interest (step 100), for example the cardiac region. Where, for example, the coronary arteries are to be examined, the guiding catheter is inserted so that it is adjacent the opening of the coronary arteries (step 110). An angioplasty guide wire is then inserted into the coronary artery, past the point of specific interest. The guide wire is usually inserted with the aid of standard fluoroscopic techniques, as is the guide catheter.

The guide catheter, when in place over the entrance to the coronary (or other target) artery will protrude a distance from the patient once in place. This is then fixed to the guide catheter extension 27. The guide catheter extension will be fixed to the guide catheter by inserting the non-compressible tube 30 into the Y-piece 31. The gland nut 32 and o-ring seal (compression fitting) is tightened to seal the joint between the guide catheter and guide catheter extension and a securing means 33 is provided which holds the Y-piece in place relative to the guide catheter extension. Alternatively, the outside surface of the non-compressible tube may be profiled with shallow circumferential grooves, to ensure that the tube will not pull out when held in the compression fitting of the Y-piece (not shown).

A seal element 34 is provided within the guide catheter extension. This is sandwiched between the non-compressible tube and the guide catheter extension body. This provides a sealing engagement between the nobn-compressible tube and the catheter.

Once the guide catheter, guide catheter extension and guide wire are in position, the catheter 5 of the present invention is maneuvered over the guide wire to a position beyond the specific area of interest in the coronary artery (step 120) with the aid of fluoroscopy. The catheter is then fixed in position on the pull-back device by clipping into mounts B and C. The guide catheter extension is then fixed in position on the mount A, at a fixation point along its length which optimises the distance between mount A and B and C. Thus, the guide catheter extension should be fixed to mount A so that the catheter may be mounted on mounts B and C in a closed configuration.

An angiogram is taken (step 130) to assess the position of the catheter in the vascular tissue. This image is saved and the position of the catheter is marked on the image so as to define a starting point for the controlled pull-back step.

The sheath 21 is then be retracted to allow the projections to adopt the deployed configuration. This is achieved by moving mount B towards mount C (usually manually). Mount C at this time is locked relative to mount A. Once the sheath 21 is retracted sufficiently to allow expansion of the resiliently biased projections, mount B is locked in position and mount C is pulled back by the drive mechanism until the projections are housed in the sheath. This is feasible if the sheath 21 is retracted sufficiently (equal or greater than the length of the pull-back distance during which measurement takes place) to allow the intermediate/central lumen 26 to be retracted in the sheath 21 without the sheath impacting on the projections along the length of measurement

Alternatively, the mount B and C are locked in position once the catheter is in the deployed configuration and both mounts are pulled back by the drive mechanism.

The locking mechanism includes a stopper rod 35. This is provided with graduations capable of engaging electrically actuated locking pins (not shown) within mounts B and/or C. A similar set of electrically actuated locking pins (not shown) within the same mounts are used to selectively connect the mounts to the drive rod 25. A set of locking pins on any particular mount may not be connected to both the drive rod 25 and the stopper rod 35 simultaneously. Thus, each mount is either in drive or stop mode. Alternatively a ratchet mechanism may be provided as the locking mechanism.

When the mount C is in drive mode, it moves relative to mount A and B. Mount C cannot be moved towards mount B when attached to the pull-back device.

The catheter may be marked to indicate when the sensors are in a deployed or in a retracted position. This may be achieved by provision of a telescopic tubing 23 with appropriate indicators or by simply marking the extreme deployed or retracted position on the apparatus.

Controlled pull-back of the catheter then takes place (step 140). The pull-back takes place at a constant speed and is controllable by the user. Pull-back topically takes place at speeds of 0.1 to 2 mm in divisions of 0.1 mm or so.

The pull-back takes place over a distance of the vascular tissue being measured. Capacitance and/or temperature readings may be taken intermittently or substantially continuously. The data transmitted by the detectors from the vascular wall is captured for data and image processing (step 150) together with a fluoroscopy/IVUS image frame.

As the catheter is withdrawn inside the artery, the projections automatically adjust their angle following the wall's morphology without losing the desired contact. The result is that the contact between the projections and the wall is continuously maintained, even when the catheter is crossing very irregular plaque formations.

Once the pull-back has been completed, the central/intermediate lumens are retracted such that the projections are withdrawn into the sheath 21 in order to place the sensors and detectors in the retracted configuration. This restores the original smooth profile of the catheter. The catheter may then be detached from the pull-back device and withdrawn from the patient or may be reinserted into the same or another blood vessel in order to take another reading. Alternatively, the catheter may be reinserted in order to enable a therapeutic or surgical intervention.

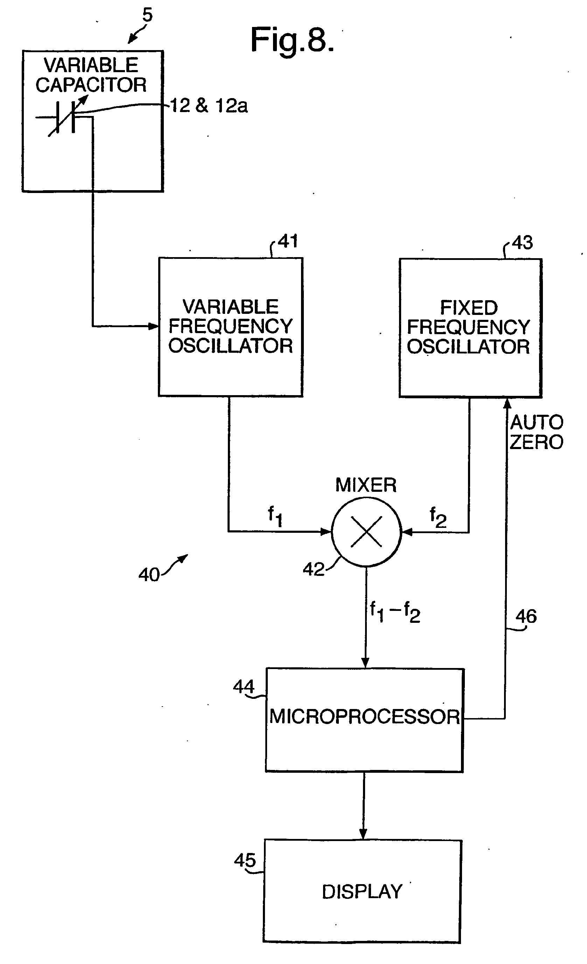

An example of a signal processing system 40 for use with the catheter 5 is shown schematically in Figure 8. Each signal channel includes a variable frequency oscillator 41 connected to a respective one of the plates 12 and 12a at the distal tip of the catheter 5. When there is an alteration of arterial wall morphology, ie a lesion effective capacitance between a plate 12 and the adjacent plate 12a will vary, thereby changing the output frequency f₁ of the associated variable frequency oscillator 41. The output f₁ of the variable frequency oscillator 41 is fed to a mixer 42 where it is mixed with the output frequency f₂ of a fixed frequency oscillator 43 to produce sum (f₁+f₂) and difference (f₁-f₂) frequencies. The fixed frequency oscillator 43 may be common to each channel. The sum frequencies are typically filtered out to leave the difference frequencies, which are fed to a microprocessor based signal processor 44 for analysis and subsequent display 45. The difference frequencies are typically in the RF range of 0-20 KHz.

The microprocessor based signal processor 44 incorporates software that implements a number of different forms of signal analysis. This may include a spectrum analyser (not shown) which analyses each signal channel and provides correlation between different channels. This data can be used to generate views of the vessel wall to indicate morphology and areas of compositional interest.

In operation, it is necessary to insert the catheter 5 and position it at a desired location. The system must then be calibrated so that the difference frequency (f₁-f₂) is detected to be zero. This is achieved by tuning the output frequency f₂ of the fixed frequency oscillator 43 by a small amount using an associated phase locked loop control mechanism (not shown). As indicated, this can be performed automatically using a feedback control loop 46. Once the system is correctly calibrated, a controlled pullback (or insertion) of the catheter 5 can be initiated to bring it into the region of interest. Depending on the configuration of the array of metallic plates 12, data can be logged automatically whilst the catheter 5 remains stationary, or alternatively the catheter 5 can be moved continuously over a length of the vessel of interest.

## Claims

1. A catheter comprising a resiliently biased projection (11) and a detector (12, 12a) which generates a signal which varies as a function of radial displacement of the resiliently biased projection relative to the longitudinal axis of the catheter, **characterised in that** the detector is a variable capacitor.

2. A catheter according to claim 1, wherein one plate of the variable capacitor (12, 12a) is mounted or formed integrally with the resiliently blased projection (11).

3. A catheter according to claim 2, wherein the capacitor plate (12, 12a) is located on the inner face of the projection (11) relative to the body of the catheter.

4. A catheter according to any preceding claim, wherein more than one projection (11) is provided and each projection is Independently biased.

5. A catheter according to any preceding claim, comprising 2 or more detectors (12, 12a), preferably 2 to 10 detectors, more preferably 2 to 6 detectors may be utilised in the present invention.

6. A catheter according to any preceding claim, wherein each detector (12, 12a) is mounted on a separate projection (11).

7. A catheter according to any preceding claim, wherein the projection (11) comprises a superelastic material, preferably nitinol.

8. A catheter according to any preceding claim, wherein the resiliently biased projection (11), when deployed, adopts an arcuate shape along at least part of its length.

9. A catheter according to any preceding claim, additionally comprising a signal processing system electrically coupled to the detector, which is adapted to detect changes in the signal of the detector.

## Patentansprüche

1. Katheter umfassend einen elastisch vorgespannten Vorsprung (11) und einen Detektor (12, 12a), der ein Signal erzeugt, das als Funktion eines radialen Versatzes des elastisch vorgespannten Vorsprungs relativ zur Längsachse des Katheters variiert,
**dadurch gekennzeichnet, dass**
der Detektor ein variabler Kondensator ist.

2. Katheter nach Anspruch 1, bei dem eine Platte des variablen Kondensators (12, 12a) befestigt ist oder mit dem elastisch vorgespannten Vorsprung (11) integral ausgebildet ist.

3. Katheter nach Anspruch 2, bei dem die Kondensatorplatte (12, 12a) an der Innenseite des Vorsprungs (11) relativ zum Körper des Katheters angeordnet ist.

4. Katheter nach einem der vorstehenden Ansprüche, bei dem mehr als ein Vorsprung (11) bereitgestellt ist und jeder Vorsprung unabhängig vorgespannt ist.

5. Katheter nach einem der vorstehenden Ansprüche umfassend 2 oder mehr Detektoren (12, 12a), bevorzugt 2 bis 10 Detektoren, wobei besonders bevorzugt 2 bis 6 Detektoren in der vorliegenden Erfindung verwendet werden können.

6. Katheter nach einem der vorstehenden Ansprüche, bei dem jeder Detektor (12, 12a) an einem separaten Vorsprung (11) befestigt ist.

7. Katheter nach einem der vorstehenden Ansprüche, bei dem der Vorsprung (11) ein superelastisches Material, bevorzugt Nitinol umfasst.

8. Katheter nach einem der vorstehenden Ansprüche, bei dem der elastisch vorgespannte Vorsprung (11), wenn eingesetzt, eine gebogene Form entlang wenigstens eines Teils seiner Länge annimmt.

9. Katheter nach einem der vorstehenden Ansprüche, zusätzlich umfassend ein Signalverarbeitungssystem, das elektrisch mit dem Detektor gekoppelt ist, das ausgelegt ist, um Veränderungen in dem Signal des Detektors zu ermitteln.

## Revendications

1. Cathéter comprenant une saillie écartée élastiquement (11) et un détecteur (12, 12a), qui génère un signal qui varie en fonction du déplacement radial de la saillie écartée élastiquement par rapport à l'axe longitudinal du cathéter, **caractérisé en ce que** le détecteur est un condensateur variable.

2. Cathéter selon la revendication 1, dans lequel une plaque du condensateur variable (12, 12a) est montée ou formée d'une pièce avec la saillie écartée élastiquement (11).

3. Cathéter selon la revendication 2, dans lequel la plaque de condensateur (12, 12a) est localisée sur la face interne de la saillie (11) par rapport au corps du cathéter.

4. Cathéter selon l'une quelconque des revendications précédentes, dans lequel plus d'une saillie (11) sont prévues et chaque saillie est indépendamment écartée.

5. Cathéter selon l'une quelconque des revendications précédentes, dans lequel 2 détecteurs (12, 12a) ou plus, de préférence 2 à 10 détecteurs, de manière plus préférée 2 à 6 détecteurs peuvent être utilisés dans la présente invention.

6. Cathéter selon l'une quelconque des revendications précédentes, dans lequel chaque détecteur (12, 12a) est monté sur une saillie (11) séparée.

7. Cathéter selon l'une quelconque des revendications précédentes, dans lequel la saillie (11) se compose d'un matériau super-élastique, de préférence le nitinol.

8. Cathéter selon l'une quelconque des revendications précédentes, dans lequel la saillie écartée élastiquement (11), lorsqu'elle est déployée, adopte une forme arquée sur au moins une partie de sa longueur.

9. Cathéter selon l'une quelconque des revendications précédentes, comprenant en outre, un système de traitement de signal, couplé électriquement au détecteur, qui est approprié pour détecter les changements de signal du détecteur.
